# EUROPEAN PATENT APPLICATION

(11) **EP 1 502 542 A1**
(43) Date of publication of application: **02.02.2005**
(21) Application number: 04254582.2
(22) Date of filing: 30.07.2004
(51) Int. Cl.: A61B 5/042, A61B 18/14, A61N 1/05

(54) **Catheter with electrode strip**

(30) Priority: 01.08.2003 US 633298
(71) Applicant: Biosense Webster, Inc., Diamond Bar, California 91765 (US)
(72) Inventor: Govari, Assaf, Haifa 34400 (IL); Altmann, Andrea Claudio, Haifa 34614 (IL)
(74) Representative: Mercer, Christopher Paul

(57) **Abstract**

Apparatus (30) for medical treatment or diagnosis in a body cavity (24) of a mammalian subject (26) includes an elongate probe (22), having an outer surface and comprising a distal portion (30), which is adapted for insertion into the body cavity. An electrode strip (32) includes an elongate insulating substrate (48), which is wrapped around the distal portion of the probe so as to define a helix having distal and proximal ends and a length therebetween, the substrate being fixed to the outer surface of the probe over substantially all of the length of the helix. A plurality of electrodes (34) are disposed along the length of the helix and fixed to the substrate. Electrical conductors (50) are coupled to the electrodes and run along the substrate over the length of the helix so as to communicate with circuitry (28) in a location proximal to the distal portion of the probe.

## Description

### FIELD OF THE INVENTION

The present invention relates generally to invasive medical devices, and specifically to devices for mapping electrical activity in the heart.

### BACKGROUND OF THE INVENTION

Catheters with electrode arrays on their outer surfaces are known in the art. For example, U.S. Patent 6, 063,022 describes a catheter with an array of electrophysiological sensing electrodes spaced along its length. The catheter also comprises position sensors, for use in determining the location of the electrodes within the body. The electrodes and position sensors can thus be used to generate a map of physiological activity as a function of position within the body cavity. In another embodiment described in this patent, the catheter comprises an array of radio frequency (RF) ablation electrodes.

Typically, in order to produce an electrode array on the catheter, a set of wires is threaded through a lumen of the distal portion of the catheter, and each of the electrodes is electrically coupled to a respective one of the wires. Assembly of such catheters is generally an expensive, labor-intensive process, which typically includes: (a) forming holes in the shaft of the catheter at the location of each electrode; (b) threading a set of wires through a lumen in the distal portion of the catheter; (c) manually drawing each wire through a respective hole in the shaft; (d) attaching each wire to a respective electrode; (e) pulling each wire back into the shaft; and (f) gluing each electrode to the outer surface of the shaft over its respective hole.

Some catheters carry electrode arrays that can be expanded when the catheter is inside a chamber of the heart, in order to enable rapid mapping of electrical activity or RF ablation in the chamber. For example, U.S. Patent 5,279,299 describes a catheter having an expandable device, which is secured to the distal extremity of the catheter and is movable between a contracted position and an expanded position. The electrodes are mounted on the expandable device so that when the expandable device is moved to the expanded position in a chamber of the heart, the electrodes are moved into engagement with the wall of the chamber. In one embodiment, the expandable element has the form of a single flexible elongate strip, which is wrapped in a spiral fashion around the catheter and is movable between contracted and expanded positions.

Other catheters use strip electrodes, rather than arrays of individual electrodes, on their outer surface. For example, U.S. Patent 6,090,104 describes a catheter having at least one spirally wrapped flat ribbon electrode. Each such electrode has an associated lead wire that can be connected to a source of energy for ablation or connected to a recording system to produce electrophysiological signals for diagnosis. The catheter is steerable by use of a puller wire connected to the distal section of the catheter and connected to a handle with means for controlling the movement of the puller wire.

### SUMMARY OF THE INVENTION

Embodiments of the present invention provide improved means and methods for fixing an electrode array to the distal portion of an invasive probe, such as a catheter. An electrode strip is wound in a helix around a distal portion of the probe and is fixed to the outer surface of the probe over substantially the entire length of the helix. The strip comprises an insulating substrate, with electrodes disposed along the length of the substrate. Electrical conductors running along the substrate couple the electrodes to circuitry inside the probe or to wires in the probe that connect to circuitry outside the proximal end of the probe.

The use of the electrode strip in this manner makes it possible to attach an array of electrodes to the probe simply and economically, without the need to create multiple holes in the probe or to run a wire to each electrode, as in devices known in the art. In embodiments of the present invention, only a single hole is typically made in the probe, for connecting the conductors at the proximal end of the electrode strip to the wires or circuits inside the probe.

Typically, the distal portion of the probe is bendable, generally for purposes of steering the probe inside the body. Bending the catheter can exert tensile and shear forces on the strip at the outside of the bend. Since the electrodes and conductors on the electrode strip are generally inelastic, these tensile forces could cause damage to the strip, such as loss of electrical contact with the electrodes. To avoid this problem, in some embodiments of the present invention, at least the distal portion of the probe comprises a relatively soft, elastic material, while the substrate of the electrode strip is strong and substantially inelastic. When the probe bends, the pressure exerted on the probe by the electrode strip at the outside of the bend causes substantial deformation of the elastic material. The tensile and shear forces exerted on the electrode strip are thus substantially reduced.

There is therefore provided, in accordance with an embodiment of the present invention, apparatus for medical treatment or diagnosis in a body cavity of a mammalian subject, the apparatus including:
an elongate probe, having an outer surface and including a distal portion, which is adapted for insertion into the body cavity; and
an electrode strip, including:
   an elongate insulating substrate, which is wrapped around the distal portion of the probe so as to define a helix having distal and proximal ends and a length therebetween, the substrate being fixed to the outer surface of the probe over substantially all of the length of the helix;
   a plurality of electrodes, disposed along the length of the helix and fixed to the substrate; and
   electrical conductors, coupled to the electrodes and running along the substrate over the length of the helix so as to communicate with circuitry in a location proximal to the distal portion of the probe.

Typically, the distal portion of the probe is adapted to bend and includes an elastic material, which substantially deforms due to a pressure exerted thereon by the electrode strip when the distal portion is bent, while the electrode strip is substantially inelastic, so that the electrode strip does not substantially deform due to a tensile force exerted thereon when the distal portion is bent. In a disclosed embodiment, the apparatus includes a glue applied between the substrate and the outer surface of the probe so as to fix the substrate to the probe, wherein the glue is sufficiently elastic so as to accommodate a relative motion between the electrode strip and the outer surface when the distal portion is bent.

In some embodiments, the substrate includes a flexible circuit substrate, and the electrodes and conductors are printed on the substrate by a printed circuit fabrication process. In one embodiment, the substrate has an inner side, which is fixed to the outer surface of the probe, and an outer side, upon which the electrodes are disposed, and the conductors are disposed along the inner side of the substrate. In another embodiment, the conductors are disposed along the outer side of the substrate.

Typically, the probe includes a cable passing therethrough in communication with the circuitry, and the conductors are coupled to the cable at the proximal end of the helix. In one embodiment, the probe includes a multiplexer, coupled between the conductors and the cable so as to select the electrodes to be coupled to the cable.

In some embodiments, the electrodes are spaced substantially evenly over the length of the helix, while in other embodiments, the electrodes are grouped in two or more clusters over the length of the helix.

In one embodiment, the probe includes a catheter, which is adapted to be inserted into a chamber of a heart of the subject. Typically, the electrodes are adapted to sense electrical signals within a wall of the heart, and the conductors are adapted to convey the signals to the circuitry. Alternatively, the electrodes are adapted to receive electrical energy from the conductors and to apply the electrical energy to a wall of the heart.

There is also provided, in accordance with an embodiment of the present invention, a method for producing a medical device, the method including:
providing an elongate probe, which is adapted for insertion into the body cavity;
wrapping an electrode strip around the probe so as to define a helix having distal and proximal ends and a length therebetween, the strip including an elongate insulating substrate having a plurality of electrodes fixed thereto and disposed along the length of the helix and further having electrical conductors, coupled to the electrodes, running along the substrate over the length of the helix so as to communicate with circuitry associated with the probe; and
fixing the substrate to an outer surface of the probe over substantially all of the length of the helix.

There is additionally provided, in accordance with an embodiment of the present invention, a method for medical diagnosis, including:
inserting an elongate probe into a body cavity of a mammalian subject, the probe having an elongate insulating substrate wrapped around a distal portion of the probe so as to define a helix having distal and proximal ends and a length therebetween, the substrate being fixed to an outer surface of the probe over substantially all of the length of the helix, wherein a plurality of electrodes are disposed along the length of the helix and fixed to the substrate, and wherein electrical conductors are coupled to the electrodes and run along the substrate over the length of the helix;
disposing the probe in the body cavity so that the electrodes sense electrophysiological activity within the cavity; and
receiving and processing signals from the electrodes via the conductors.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a cardiac catheterization system, in accordance with an embodiment of the present invention;
Fig. 2 is a schematic side view of a distal portion of a catheter with an electrode strip fixed thereto, in accordance with an embodiment of the present invention;
Fig. 3 is a schematic, pictorial view of an electrode strip, in accordance with an embodiment of the present invention;
Fig. 4 is a schematic cutaway view of a heart with a catheter inserted therein, in accordance with an embodiment of the present invention;
Fig. 5 is a schematic frontal view of an electrode strip, in accordance with an embodiment of the present invention;
Fig. 6 is a schematic, sectional view of a portion of a catheter having an electrode strip fixed thereto, in accordance with an embodiment of the present invention;
Fig. 7 is a schematic frontal view of an electrode strip, in accordance with another embodiment of the present invention; and
Fig. 8 is a block diagram that schematically shows multiplexing circuitry inside a catheter, in accordance with an embodiment of the present invention.

### DETAILED DESCRIPTION OF EMBODIMENTS

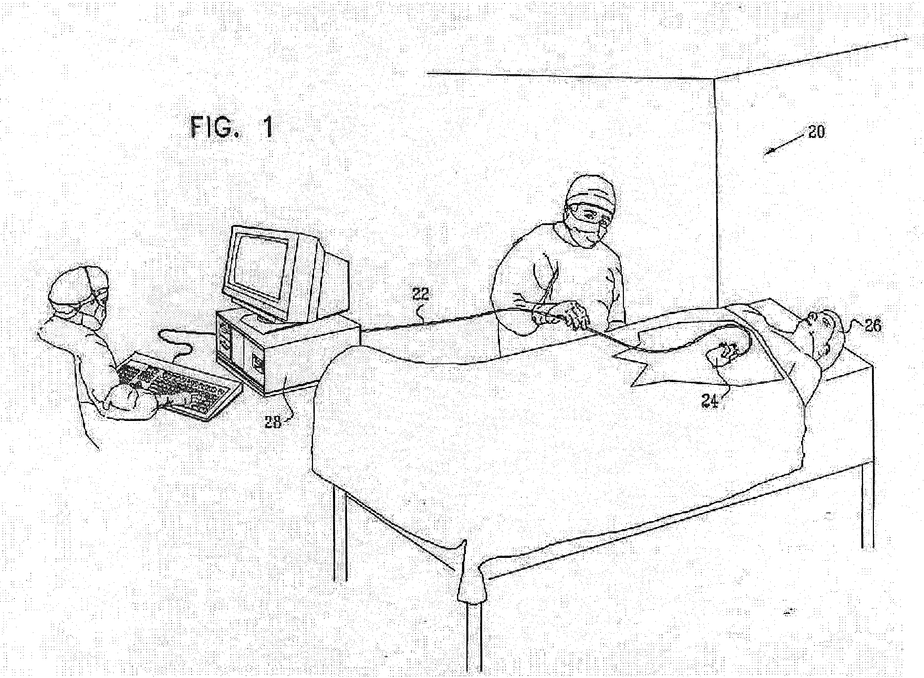

Fig. 1 is a schematic, pictorial illustration of a cardiac catheterization system 20, in accordance with an embodiment of the present invention. System 20 comprises an elongate probe, typically a catheter 22, which is inserted by a user through a vein or artery of a human or other mammalian subject 26 into a chamber of a heart 24 of the subject. Catheter 22 is coupled at its proximal end to a console 28, which receives electrical signals from electrodes fixed to the distal end of the catheter inside the heart, as described hereinbelow. The console may use these signals to create a map of electrical activity in the heart, as is known in the art. Alternatively or additionally, the console may be configured to provide electrical energy, typically RF energy, to the electrodes in order to ablate areas of the endocardium, as is likewise known in the art.

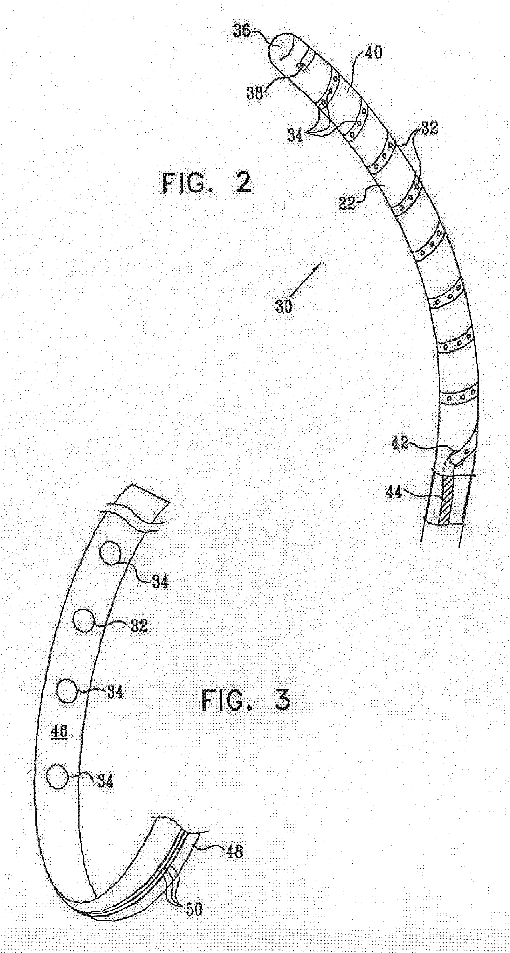

Fig. 2 is a schematic side view of a distal portion 30 of catheter 22, in accordance with an embodiment of the present invention. An electrode strip 32 is wound in a helix around the distal portion of the catheter. The electrode strip comprises an array of electrodes 34, which are electrically exposed on the outer surface of the strip. The strip typically has a width of about 2 mm, a length between about 10 cm and about 12 cm, and a thickness of about 0.03 mm. Typically, there are about twenty-five electrodes 34 on the strip. Alternatively, electrode strips of this sort may be produced in larger or smaller sizes, and with greater or smaller numbers of electrodes. Catheter 22 may comprise other elements in distal portion 30, which are not shown in the figures, including a steering mechanism and sensors of other types, such as position sensors. Such elements are described, for example, in the above-mentioned U.S. Patent 6,063,022.

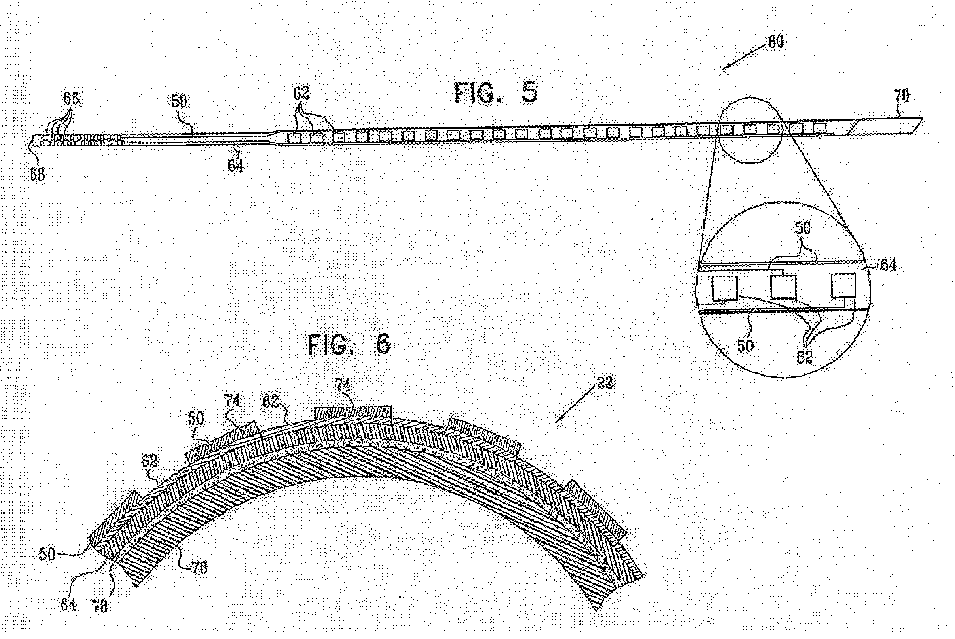

Fig. 3 is a schematic pictorial illustration of a segment of electrode strip 32, showing portions of both an outer side 46 and an inner side 48 of the strip, in accordance with an embodiment of the present invention. Strip 32 comprises a micro-flex circuit, produced on a flexible, non-conductive substrate, typically a biocompatible plastic, such as polyimide. Electrodes 34 are deposited on the outer side of the substrate, typically using methods of printed circuit production known in the art. The electrodes are connected through the substrate to conductive traces 50 on inner side 48 of strip 32. The traces are typically arranged such that each of the traces is electrically coupled to exactly one of the electrodes on the opposite side of the strip. Traces 50 are typically about 11 µm wide and 1 µm thick, on 22 µm centers. The traces may be formed near the center line of strip 32 in order to minimize shear forces on the traces. Further details of the construction of strip 32 are shown below in Figs. 5, 6 and 7.

Returning now to Fig. 2, in order to assemble catheter 22, the distal end of electrode strip 32 is secured to distal portion 30 of catheter 22 in the vicinity of a distal tip 36 of the catheter. The strip may be secured, for example, by using a fastener 38, such as a pin or screw, or by gluing its distal end to the catheter. Strip 32 is then spirally wrapped tightly about distal portion 30 of the catheter, and is permanently secured thereto along the length of the strip, by means such as glue. The proximal end of the electrode strip is inserted into catheter 22 through an aperture 42 (which is subsequently sealed). Inside the catheter, traces 50 are electrically coupled to a cable 44 or other signal transfer medium, which connects at the proximal end of catheter 22 to console 28. Cable 44 may comprise, for example, a MicroFlat ribbon cable (produced by W.L. Gore & Associates, Elkton, Maryland), which contains individual wires having a one-to-one correspondence with traces 50. Alternatively, multiple traces may be multiplexed onto a single wire, as described hereinbelow with reference to Fig. 8.

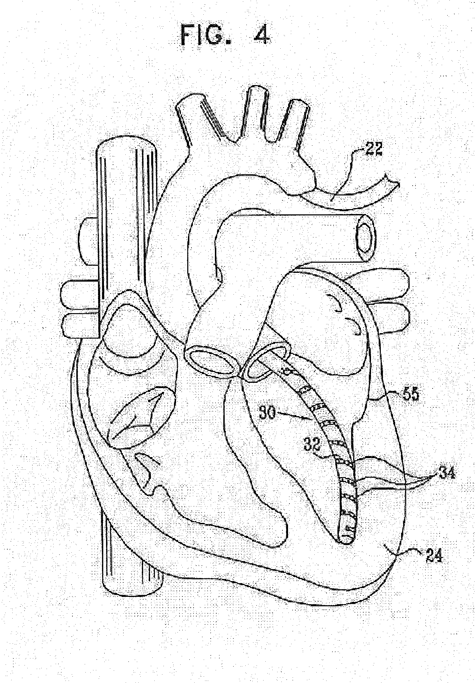

Fig. 4 is a schematic, cutaway illustration of heart 24, showing distal portion 30 of catheter 22 inserted inside a chamber 55 of the heart, in accordance with an embodiment of the present invention. The distal portion of the catheter is brought into contact with the inner wall of chamber 55, causing electrodes 34 on strip 32 to receive electrical signals from the myocardium. Alternatively, electrodes 34 may be configured to receive electrical signals within chamber 55 without physically contacting the heart wall, as described, for example, in U.S. Patent 6,400,981.

Fig. 5 is a schematic frontal view of an electrode strip 60, in accordance with an embodiment of the present invention. This strip may be used interchangeably with strip 32, shown in the preceding figures. Strip 60 comprises electrode pads 62 formed on a polyimide substrate 64. The substrate is typically about 1.8 mm wide and 12.5 µm thick. The electrode pads themselves are about 1.3 x 1.5 mm across, and are spaced about 1.4 mm apart. The pads are fabricated on the substrate by methods of flexible printed circuit production known in the art. The pads may be produced, for example, by depositing a thin layer of nickel chromium (typically about 0.5 nm thick), overlaid by about 1 µm of gold. To reduce the impedance of the electrodes, pads 62 may be plated with a variety of materials, as are known in the art, such as platinum, platinum black, iridium oxide, activated iridium, or titanium nitride. It will be understood, however, that all the dimensions and materials cited here are provided by way of example, and other materials, dimensions and methods for construction of electrode strips will be apparent to those skilled in the art.

Traces 50 are printed on substrate 64 and connect electrode pads 62 to corresponding contact pads 66, at a proximal end 68 of strip 60. The traces in this embodiment are printed on the same (outer) side of the substrate as are the electrode pads, passing along the margins of the substrate outside pads 62, as shown in the enlarged inset in Fig. 5. In order to maximize the available area of pads 62, without making strip 60 any wider than necessary, traces are preferably very narrow, typically on the order of 10 µm wide. Typically, end 68 is inserted into catheter 22, and contact pads 66 are used for connecting the traces to cable 44, as described above. A distal end 70 of strip 60 may be strengthened for secure fastening to distal portion 30 of catheter 22.

Fig. 6 is a schematic, sectional view of catheter 22, showing a detail of distal portion 30 of the catheter with electrode strip 60 fixed thereto, in accordance with an embodiment of the present invention. As noted above, in this illustration, traces 50 are formed alongside electrode pads 60 on the outer surface of substrate 64. The traces are overlaid by an additional protective layer 74, such as another 12.5 µm layer of polyimide. Thus, the total thickness of strip 60 is about 26 µm. Assuming the radius of catheter is about 1 mm, the ratio of the radius of curvature of strip 60 to its thickness is about 40. Alternatively, traces 50 may be printed on the inner surface of substrate 64, as described above. For the sake of visual clarity, the dimensions in Fig. 6 are not shown to scale. It will be understood in any case that the dimensions given above are provided solely by way of example, and larger or smaller dimensions may similarly be used, depending on application requirements and material characteristics.

Strip 60 is wrapped tightly around an outer wall 76 of catheter 22, and is fastened to wall 76 along substantially the entire length of the strip, typically by a layer of medical-grade glue 78. For example, glue 78 may comprise a two-part polyurethane mix, such as a mixture of Vorite® 689 and Polycin® 640-M1 (produced by G.R. O'Shea, Itasca, Illinois). The inventors found that a mixture of 81.8:100 (Polycin:Vorite) of these materials gave satisfactory results. Alternatively, a cyanoacrylic or urethane acrylate adhesive, such as 201-CTH (Dymax Corporation, Torrington, Connecticut) may be used. Substrate 64 of strip 60 typically has a high tensile strength, which may be on the order of 400,000 psi, and a high Young's modulus, so that the strip resists stretching or breaking when subjected to tensile or shear forces. Such forces may be generated when catheter 22 is bent, as shown in Fig. 4, particularly on the outside of the bend. If strip 60 were sufficiently elastic to stretch under these forces, conductors 50 or electrodes 62 might tear or suffer other damage.

In order to reduce the tensile force exerted on strip 60, wall 76 may be formed of an elastic material, such as a suitable medical-grade polyurethane or PVC. For example, the wall may be made from a PELLETHANE thermoplastic polyurethane elastomer (Dow Chemical, Midland, Michigan). Such a wall material is soft enough to deform inward under the pressure exerted thereon by the portion of strip 60 that is on the outside of a bend in the catheter. Glue 78 preferably has high tensile strength, as well (typically at least 1,500 psi), to avoid detachment of substrate 64 from wall 76 when the catheter bends. Unlike the substrate, the glue may be chosen to allow stretching of the glue layer, typically by up to about 175%, under the shear force that is exerted between substrate 64 and wall 76.

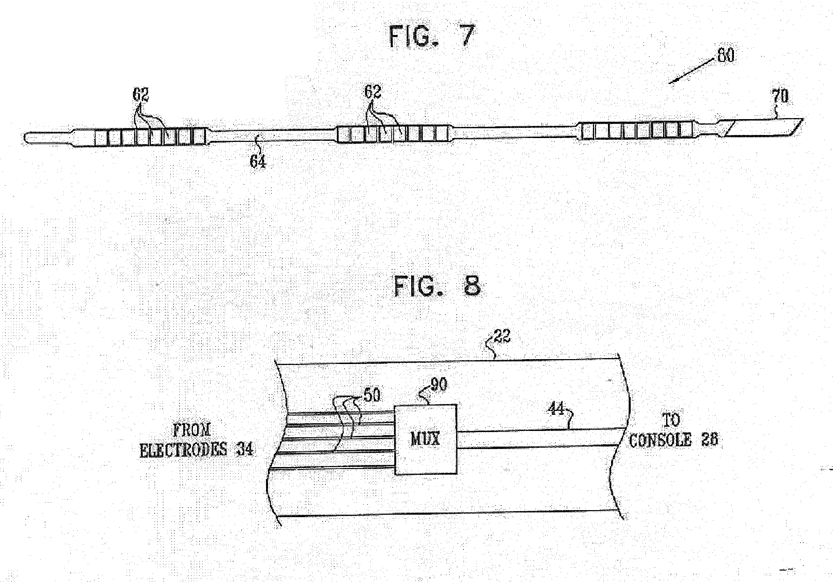

Fig. 7 is a schematic frontal view of an electrode strip 80, in accordance with another embodiment of the present invention. In this embodiment, electrodes 62 are clustered in groups along the length of substrate 64, rather than being evenly distributed as in Fig. 5. The strip characteristics illustrated in Figs. 3, 5 and 7 are shown here solely by way of example, and other electrode configurations, shapes and sizes may also be used, as will be apparent to those skilled in the art.

Fig. 8 is a block diagram that schematically illustrates a multiplexer 90 in catheter 22, for connecting traces 50 to cable 44, in accordance with an embodiment of the present invention. The use of the multiplexer reduces the number of wires that must be passed through catheter 22 to console 28, thereby allowing the catheter to be made thinner and more flexible, or leaving room to accommodate other functional elements inside the catheter. Multiplexer 90 may comprise an analog/digital converter, which converts the electrode signals on traces 50 to digital samples. In this case, the multiplexer may also comprise a digital multiplexer, using substantially any suitable digital multiplexing technique, such as time division, frequency division, or code division multiplexing. Alternatively, multiplexer 90 may comprise analog multiplexing circuitry, such as a switch, for selecting the signals from traces 50 to be conveyed over cable 44 at any given time. When multiplexer 90 is used, cable 44 typically comprises about five to seven wires, as opposed to the much larger number of wires that would be required otherwise. Thus, there has been described a method for medical diagnosis, comprising:
inserting an elongate probe into a body cavity of a mammalian subject, the probe having an elongate insulating substrate wrapped around a distal portion of the probe so as to define a helix having distal and proximal ends and a length therebetween, the substrate being fixed to an outer surface of the probe over substantially all of the length of the helix, wherein a plurality of electrodes are disposed along the length of the helix and fixed to the substrate, and wherein electrical conductors are coupled to the electrodes and run along the substrate over the length of the helix;
disposing the probe in the body cavity so that the electrodes sense electrophysiological activity within the cavity; and
receiving and processing signals from the electrodes via the conductors.

Preferably, in the method, inserting the elongate probe comprises inserting a catheter into a chamber of a heart of the subject.

Although the fabrication and use of electrode strips are described hereinabove mainly with reference to cardiac catheter 22, the principles of the present invention may similarly be applied to elongate probes that are used in examining and treating other body organs and cavities, as well. It will thus be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. Apparatus for medical treatment or diagnosis in a body cavity of a mammalian subject, the apparatus comprising:
an elongate probe, having an outer surface and comprising a distal portion, which is adapted for insertion into the body cavity; and
an electrode strip, comprising:
an elongate insulating substrate, which is wrapped around the distal portion of the probe so as to define a helix having distal and proximal ends and a length therebetween, the substrate being fixed to the outer surface of the probe over substantially all of the length of the helix;
a plurality of electrodes, disposed along the length of the helix and fixed to the substrate; and
electrical conductors, coupled to the electrodes and running along the substrate over the length of the helix so as to communicate with circuitry in a location proximal to the distal portion of the probe.

2. The apparatus according to claim 1, wherein the distal portion of the probe is adapted to bend and comprises an elastic material, which substantially deforms due to a pressure exerted thereon by the electrode strip when the distal portion is bent.

3. The apparatus according to claim 2, wherein the electrode strip is substantially inelastic, so that the electrode strip does not substantially deform due to a tensile force exerted thereon when the distal portion is bent.

4. The apparatus according to claim 3, and comprising a glue applied between the substrate and the outer surface of the probe so as to fix the substrate to the probe, wherein the glue is sufficiently elastic so as to accommodate a relative motion between the electrode strip and the outer surface when the distal portion is bent.

5. The apparatus according to claim 1, wherein the substrate comprises a flexible circuit substrate, and wherein the electrodes and conductors are printed on the substrate by a printed circuit fabrication process.

6. The apparatus according to claim 5, wherein the substrate has an inner side, which is fixed to the outer surface of the probe, and an outer side, upon which the electrodes are disposed, and wherein the conductors are disposed along the inner side of the substrate.

7. The apparatus according to claim 5, wherein the substrate has an inner side, which is fixed to the outer surface of the probe, and an outer side, upon which the electrodes are disposed, and wherein the conductors are disposed along the outer side of the substrate.

8. The apparatus according to claim 1, wherein the probe comprises a cable passing therethrough in communication with the circuitry, and wherein the conductors are coupled to the cable at the proximal end of the helix.

9. The apparatus according to claim 7, wherein the probe comprises a multiplexer, coupled between the conductors and the cable so as to select the electrodes to be coupled to the cable.

10. The apparatus according to claim 1, wherein the electrodes are spaced substantially evenly over the length of the helix.

11. The apparatus according to claim 1, wherein the electrodes are grouped in two or more clusters over the length of the helix.

12. The apparatus according to claim 1, wherein the probe comprises a catheter, which is adapted to be inserted into a chamber of a heart of the subject.

13. The apparatus according to claim 12, wherein the electrodes are adapted to sense electrical signals within a wall of the heart, and wherein the conductors are adapted to convey the signals to the circuitry.

14. The apparatus according to claim 12, wherein the electrodes are adapted to receive electrical energy from the conductors and to apply the electrical energy to a wall of the heart.

15. A method for producing a medical device, the method comprising:
providing an elongate probe, which is adapted for insertion into the body cavity;
wrapping an electrode strip around the probe so as to define a helix having distal and proximal ends and a length therebetween, the strip comprising an elongate insulating substrate having a plurality of electrodes fixed thereto and disposed along the length of the helix and further having electrical conductors, coupled to the electrodes, running along the substrate over the length of the helix so as to communicate with circuitry associated with the probe; and
fixing the substrate to an outer surface of the probe over substantially all of the length of the helix.

16. The method according to claim 14, wherein the probe is adapted to bend and comprises an elastic material, which substantially deforms due to a pressure exerted thereon by the electrode strip when the probe is bent.

17. The method according to claim 16, wherein the electrode strip is substantially inelastic, so that the electrode strip does not substantially deform due to a tensile force exerted thereon when the distal portion is bent.

18. The method according to claim 17, wherein fixing the substrate comprises applying a glue between the substrate and the outer surface of the probe, wherein the glue is sufficiently elastic to accommodate a relative motion between the electrode strip and the outer surface when the distal portion is bent.

19. The method according to claim 14, wherein the substrate comprises a flexible circuit substrate, and comprising printing the electrodes and conductors on the substrate by a printed circuit fabrication process.

20. The method according to claim 19, wherein printing the electrodes and conductors comprises printing the conductors on an inner side of the substrate, which is fixed to the outer surface of the probe, and printing the electrodes on an outer side of the substrate, opposite the inner side.

21. The method according to claim 19, wherein fixing the substrate comprises fixing an inner side of the substrate to the outer surface of the probe, and wherein printing the electrodes and conductors comprises printing the electrodes and conductors on an outer side of the substrate, opposite the inner side.

22. The method according to claim 14, and comprising passing a cable through the probe, and coupling the cable to the conductors at the proximal end of the helix so as to provide a connection between the electrodes and the circuitry.

23. The method according to claim 14, wherein the probe comprises a catheter, which is adapted to be inserted into a chamber of a heart of the subject.
